# EUROPÄISCHE PATENTSCHRIFT

(11) **EP 1 372 689 B1**
(45) Veröffentlichungstag und Bekanntmachung des Hinweises auf die Patenterteilung: **14.07.2004**
(21) Anmeldenummer: 02730066.4
(22) Anmeldetag: 26.03.2002
(51) Int. Cl.: A61K 38/00, A61P 9/10

(54) **VERWENDUNG VON INHIBITOREN VON IKK-BETA UND VERFAHREN ZUM AUFFINDEN SOLCHER INHIBITOREN**
USE OF IKK-BETA INHIBITORS AND METHOD FOR DISCOVERY OF SAID INHIBITORS
UTILISATION D'INHIBITEURS D'IKK-BETA ET PROCEDE DE RECHERCHE DESDITS INHIBITEURS

(30) Priorität: 27.03.2001 DE 10115073
(43) Veröffentlichungstag der Anmeldung: 02.01.2004
(73) Patentinhaber: Procorde GmbH, 82152 Martinsried (DE)
(72) Erfinder: UNGERER, Martin, 80799 München (DE); BRAND, Korbinian, 80307 München (DE); GAWAZ, Meinrad, 80805 München (DE)
(74) Vertreter: Wächtershäuser, Günter, Prof. Dr.
(86) Internationale Anmeldenummer: PCT/EP2002/003382
(87) Internationale Veröffentlichungsnummer: WO 2002/076482

(56) Entgegenhaltungen:
- WO-A-01/10438
- WO-A-96/28168
- DE-A- 19 928 424
- US-A- 5 939 302
- US-A- 6 121 249
- YIN M-J ET AL: "THE ANTI-INFLAMMATORY AGENTS ASPIRIN AND SALICYLATE INHIBIT THE ACTIVITY OF IKAPPAB KINASE-BETA" NATURE, MACMILLAN JOURNALS LTD. LONDON, GB, Bd. 396, 5. November 1998 (1998-11-05), Seiten 77-80, XP002938591 ISSN: 0028-0836
- GAWAZ, M. (1) ET AL: "Effects of transient platelet interaction with endothelial cells on IkappaB kinase complex activation. Implications for atherogenesis." ANNALS OF HEMATOLOGY, (2001) VOL. 80, NO. SUPPLEMENT 1, PP. A19. PRINT. MEETING INFO.: 45TH ANNUAL MEETING OF THE SOCIETY FOR THROMBOSIS/HEMOSTASIS RESEARCH DUESSELDORF, GERMANY FEBRUARY 14-17, 2001 , XP008014920

## Beschreibung

Die vorliegende Erfindung betrifft eine neue Verwendung spezieller Inhibitoren zur Herstellung von Medikamenten zur Vorbeugung und Behandlung von Atherosklerose sowie ein spezielles Screeningverfahren zum Auffinden neuer Inhibitoren.

Es ist bekannt, dass Atherosklerose eine Folge von Läsionen des Gefäßendothels ist, die durch Hypertonie, lokale Wirbelbildung oder Sauerstoffmangel begünstigt werden. Man nimmt an, dass aufgrund der von den Läsionen freigelegten subendothelialen Strukturen der Gefäßwand, zirkulierende Blutplättchen an den geschädigten Stellen des Endothels anhaften (Plättchenadhäsion) und danach miteinander verklumpen (Plättchenaggregation).

Durch die Wechselwirkung der Blutplättchen mit den Endothelzellen kommt es zu einer Vielzahl von Folgeprozessen, die zu einer Akkumulation von Lipiden in der Gefäßwand führen. Mit der Akkumulation von Lipiden in der Gefäßwand beginnt die Bildung von sogenannten atherosklerotischen Plaques. In den Plaques reichern sich Schaumzellen an, die aus eingewanderten Makrophagen entstehen. Schaumzellen nehmen die akkumulierten Lipide auf und bauen diese ab. Während einige Lipoproteine einfach abgebaut werden können, bleiben Cholesterin und Cholesterinester praktisch ungespalten. Die Schaumzellen werden daher mit Cholesterin und Cholesterinestern überladen.

Als Folge von atherosklerotischen Plaques kann es zu zentralen Nekrosen kommen, in deren Nachbarschaft Kalksalze abgelagert werden. In fortgeschrittenen Stadien können Plaques einreißen, so dass atherosklerotische Läsionen entstehen, die nachfolgend von einem Thrombus abgedeckt werden. Die Verdickung der Arterienwand sowie die Bildung von oberflächlichen Thromben können das Gefäßlumen bis zum völligen Verschluss einengen. Thromben, die sich von größeren Arterien ablösen, verursachen einen Verschluss kleinerer Gefäße in der Körperperipherie. Arterielle Durchblutungsstörungen, denen in den meisten Fällen atherosklerotische Prozesse zugrundeliegen, sind mit einem Anteil von etwa 50 % die häufigste Todesursache in den sogenannten Industriestaaten.

Die molekularen Mechanismen der Atherogenese werden kaum verstanden, insbesondere im Hinblick auf Prozesse, die die Chemotaxis von Monocyten und die Transmigration von Monocyten bestimmen. Es ist jedoch bereits bekannt, dass das Cytokin MCP-1 (Monocyte Chemoattractant Protein-1) bei Enzündungsprozessen der Arterienwand auftritt und Monocyten zum Ort der Entzündung lockt. Es ist ebenfalls bekannt, dass MCP-1 in Endothelzellen exprimiert werden kann. Die Expression und Sekretion von MCP-1 wird über komplexe Signalkaskaden gesteuert. Einzelheiten der Steuerung der Expression und Sekretion von MCP-1 in Endothelzellen in frühen Stadien der Atherogenese sind aus dem Stand der Technik nicht bekannt. Insbesondere sind keine Zielproteine im Zusammenhang mit Atherosklerose bekannt, durch deren Beeinflussung das Auftreten und der frühe Verlauf der Krankheit beeinflusst werden kann.

Die Verhinderung oder Verminderung des Anlockens und der Einwanderung von Monocyten in die Gefäßwand im Rahmen von atherosklerotischen Prozessen kann die Anreicherung von Schaumzellen in atherosklerotischen Plaques verlangsamen und wäre daher geeignet, atherosklerotische Prozesse zu unterdrücken oder zu verhindern

Es ist Aufgabe der Erfindung, ein neues Verfahren bereitzustellen, mit dem Atherosklerose verhindert oder behandelt werden kann.

Es ist weiterhin Aufgabe der vorliegenden Erfindung ein Screeningverfahren zum Auffinden neuer Wirkstoffe bereitzustellen, mit denen Atherosklerose verhindert oder behandelt werden kann.

Diese Aufgaben werden gemäß den Ansprüchen gelöst. Die vorliegende Erfindung beruht auf der Erkenntnis, dass eine Expression von MCP-1 in Endothelzellen keine Blutplättchenaggregation auf dem Gefäßendothel voraussetzt. Vielmehr induzieren aktivierte Blutplättchen bereits in einem Stadium der Atherogenese eine Expression von MCP-1 in Endothelzellen, in dem noch keine Plättchenaggregation nachgewiesen werden kann. Es wurde gefunden, dass aktivierte Blutplättchen, die *in vivo* ohne zu verklumpen lediglich vorübergehend aber fest an einer durch Ischämie aktivierten endothelialen Monoschicht anhaften, degranulieren und den Transkriptionsfaktor NF-κB in den benachbarten Endothelzellen aktivieren. Diese neue Form der Wechselwirkung zwischen Blutplättchen und aktivierten Endothelzellen, die keine erkennbaren Läsionen voraussetzt, unterscheidet sich einerseits von den aus dem Stand der Technik bekannten Rolling-Wechselwirkungen bei denen es zu keiner festen Adhäsion kommt und andererseits von dem festen Anhaften und Verklumpen bei der Plättchenaggregation, das nicht nur vorrübergehend, sondern permanent ist. Die durch aktivierte Blutplättchen vermittelte Aktivierung von NF-κB in den Endothelzellen führt zu einer Sekretion von MCP-1 aus den Endothelzellen. Durch die Sekretion von MCP-1 werden in frühen Stadien der Atherogenese Monocyten angelockt, die in die Gefäßwand einwandern können. Eine lediglich vorübergehende Anhaftung von aktivierten Blutplättchen führt daher zu einer Sekretion von MCP-1, die eine Voraussetzung für die Bildung von Plaques in atherosklerotischen Prozessen ist.

Die vorliegende Erfindung beruht ferner auf der Erkenntnis, dass bei der durch aktivierte Blutplättchen vermittelten Aktivierung von NF-κB das Inhibitorprotein IκB speziell von der IKK-β-Kinase des IKK-Komplexes phosphoryliert und damit aktiviert wird. Durch die spezielle kurzzeitige Aktivierung durch aktivierte Blutplättchen kommt es unter Beteiligung von IKK-β zu einer starken und andauernden Phosphorylierungsaktivität von IκB, die bis zu einer Stunde andauert. Durch den damit verbundenen Abbau des Inhibitorproteins IκB kommt es zu einer lange andauernden und starken Aktivierung von NF-κB und einer entsprechenden Sekretion von MCP-1. Dies ist überraschend im Hinblick auf die Tatsache, dass die Wechselwirkung mit Blutplättchen nur vergleichsweise kurzzeitig erfolgt.

Das zeitbezogene Aktivierungsprofil des IKK-Komplexes bei der Aktivierung durch aktivierte Blutplättchen ist grundsätzlich verschieden von denjenigen der Aktivierung durch IL-1β oder TNF, die aus dem Stand der Technik bereits als Aktivatoren des IKK-Komplexes bekannt sind. Aktivierte Blutplättchen aktivieren den IKK-Komplex für etwa 60 Minuten, wobei das maximale Phosphorylierungsniveau nach etwa 30 Minuten erreicht wird und kaum ein Unterschied zwischen der Aktivierung von IKK-α und IKK-β beobachtet wird. TNF führt zu einer nur wenige Minuten andauernden extrem starken Aktivierung, insbesondere des IKK-Komplexes, die jedoch nach etwa 20 Minuten nicht mehr nachweisbar ist. IL-1β hingegen bewirkt eine sehr lang andauernde Aktivierung, insbesondere von IKK-β, die etwa zwei Stunden anhält. Die Aktivierung von IKK-β ist dabei aber wesentlich schwächer als die Aktivierung durch aktivierte Blutplättchen.

Durch die Aktivierung des IKK-Komplexes mit aktivierten Blutplättchen wird daher die Kinase IKK-β stark und langandauernd aktiviert, was für eine Aktivierung von NF-κB im Zusammenhang mit einer Sekretion von MCP-1 und daher für die Bildung von Plaques in atherosklerotischen Prozessen entscheidend ist. Dieser Mechanismus betrifft nicht nur einen entscheidenden Schritt in der Regulation früher atherosklerotischer Prozesse, sondern auch in der Plaquebildung, -ausbreitung und -destabilisierung.

Die Erfindung schlägt daher die Verwendung eines Inhibitors von IKK-β zur Herstellung eines Medikaments zur Behandlung von oder zur Vorbeugung gegen Atherosklerose vor.

Die Erfindung bietet den Vorteil, dass bei Inhibierung von IKK-β in einem frühen Stadium der Atherogenese die Endothelschicht noch nicht durch Plättchenaggregation oder oberflächlicher Thrombenbildung abgeschirmt ist, so dass die Endothelzellen einfach und wirksam mit Inhibitoren erreicht werden können, die eine Expression von MCP-1 beeinflussen. Dieses Stadium ist durch minimale Endothelläsionen, häufig sogenannte "fatty streaks" gekennzeichnet.

Die Erfindung bietet weiterhin den Vorteil, dass bei Inhibierung von IKK-β in Endothelzellen, die atherosklerotischen Plaques benachbart sind, eine Vergrößerung der Plaques verhindert werden kann oder bestehende Plaques stabilisiert werden können.

Die Erfindung bietet weiterhin den Vorteil, dass ein Protein in der Signalkaskade der Expression von MCP-1 inhibiert wird, das bei der Aktivierung durch aktivierte Blutplättchen eine zentrale Rolle spielt, wobei es sich bei dem Protein nicht um NF-κB handelt, dessen direkte Beeinflussung zu schweren Nebenwirkungen führen würde, weil NF-κB ebenfalls an der Expression von weiteren Proteinen beteiligt ist, wie an der Expression von Cytokinen, Chemokinen, Wachstumsfaktoren, beispielsweise TNF, IL-1β, IL-8, Adhäsionsmolekülen, beispielsweise ICAM-1, VCAM-1, und ELAM-1, Proteasen, beispielsweise MMP-9, und dem prothrombogenen Molekül TF. Vielmehr wird mit der Inhibierung von IKK-β ein Protein inhibiert, das einen speziell mit atherosklerotischen Prozessen in Zusammenhang stehenden wirkungsvollen Aktivierungsweg von NF-κB betrifft.

Die Erfindung bietet ferner den Vorteil, dass durch die Vermeidung einer langandauernden Sekretion von MCP-1 aus Endothelzellen, der Effekt bei der Vermeidung eines Einwandern von Monocyten ungleich höher ist, als bei Vermeidung einer kurzzeitigen, aber stärkeren Sekretion von MCP-1, wie im Fall einer Inhibierung von TNF.

Die Erfindung bietet außerdem den Vorteil, dass der Monozyten/Makrophagenfluss in die Gefäßwand auf dem Niveau der Endothelschicht reguliert werden kann und somit bevor die Monozyten/Makrophagen in die Gefäßwand eingedrungen sind.

Anders als bei herkömmlichen Behandlungen der Atherosklerose kann erfindungsgemäß eine neue Klasse von Patienten für eine Therapie ausgewählt werden, für die bekannte Therapien unwirksam sind oder für die bekannte Therapien im Hinblick auf die Nebenwirkungen unakzeptabel sind.

Eine erste spezielle Unterklasse von Patienten betrifft solche, die zur Verhinderung eines kardiovaskulären Ereignisses, wie Schlaganfall oder Herzinfarkt, zur Primärprophylaxe erfindungsgemäß mit einem Inhibitor von IKK-β behandelt werden, wobei diese Patienten aufgrund einer Beurteilung von Risikofaktoren und/oder aufgrund einer Diagnose eines frühen Stadiums der Atherosklerose ausgewählt und dieser ersten speziellen Unterklasse zugeordnet werden können.

Die Auswahl und Zuordnung der Patienten aufgrund einer Beurteilung von Risikofaktoren kann auf der Grundlage einer Bewertung der bekannten Risikofaktoren erfolgen. Die Bewertung der ermittelten Risikofaktoren kann subjektiv auf der Grundlage der allgemeinen Erfahrung des behandelnden Arztes oder objektiv auf der Grundlage von standartisierten Bewertungsverfahren erfolgen. Es ist bevorzugt ein standartisiertes Bewertungsverfahren einzusetzen. Insbesondere ist es bevorzugt das von G. Assmann et al. (Circulation 2002;105:310-315) auf der Grundlage der PROCAM-Studie vorgeschlagene Bewertungssystem einzusetzen. Bei diesem Bewertungssystem werden den folgenden für einen Patienten bestimmte Risikofaktoren standartisierte Koeffizienten zugeordnet aus deren Summe sich ein Wert ergibt, der mit einem Risiko für das Auftreten eines akuten kardiovaskulären Ereignisses in den folgenden zehn Jahren korreliert: Alter, LDL-Cholesterin, HDL-Cholesterin, Triglyceride, Raucher/Nichtraucher, Diabetes mellitus, Auftreten von MI in der Familie, sowie der systolische Blutdruck. Patienten mit einem Wert, der größer oder gleich 50 beträgt können ausgewählt und dieser Unterklasse zugeordnet werden.

Die Auswahl und Zuordnung der Patienten aufgrund einer Diagnose eines frühen Stadiums der Atherosklerose kann auf der Grundlage einer Bewertung der bekannten Symptome für das vorliegen von Atherosklerose erfolgen. Insbesondere kann die Auswahl auf der Grundlage einer Messung der mittleren Intima-Maxima-Wanddicke der Arteria carotis interna erfolgen, wobei eine Dicke von mehr als 1,2 mm auf eine atherosklerotische Veränderung hinweist. Die Messung der Wanddicke erfolgt mit einem Ultraschallgerät, dass eine geeignete Auflösung zuläßt, wie beispielsweise ein herkömmliches Ultraschallgerät mit einem 12 MHz-Schallkopf.

Dieser Unterklasse können auch Patienten angehören, die ohne, dass ein kardiovaskuläres Ereignis bisher aufgetreten ist, bereits lipidreiche Plaques oder gar Plaques mit Kalkeinlagerungen aufweisen.

Erfindungsgemäß kann bei dieser Unterklasse durch Verabreichung eines Inhibitors von IKK-β durch Primärprophylaxe das Fortschreiten der atherosklerotischen Veränderungen der Gefäße verhindert werden, so dass das Risiko für ein kardiovaskuläres Ereignis oder das Ansteigen des Risikos für ein kardiovaskuläres Ereignis gemindert wird.

Eine zweite spezielle Unterklasse von Patienten betrifft solche, die zur Verhinderung weiteren kardiovaskulärer Ereignisse, wie Schlaganfall oder Herzinfarkt, zur Sekundärprophylaxe nach einem kardiovaskulären Ereignis erfindungsgemäß mit einem Inhibitor von IKK-β behandelt werden. In diesem Fall können die Patienten allein aufgrund des Primärereignisses und/oder wie oben beschrieben aufgrund einer Beurteilung von Risikofaktoren und/oder aufgrund einer Diagnose der Atherosklerose ausgewählt und dieser zweiten speziellen Unterklasse zugeordnet werden.

Die vorliegende Erfindung betrifft daher auch Verfahren zur Vorbeugung oder Behandlung von Atherosklerose, das dadurch gekennzeichnet ist, dass Patienten ein Inhibitor von IKK-β verabreicht wird. Der Inhibitor kann auf alle bekannten Arten verabreicht werden. Beispielsweise kann der Inhibitor enteral oder parenteral verabreicht werden. Andererseits kann der Inhibitor auch als Vorstufe, wie beispielsweise als Vektor, durch eine Gentherapie verabreicht werden. Eine enterale Verabreichung erfolgt vorzugsweise oral. Eine parenterale Verabreichung kann intravenös, intraarteriell, intramuskulär oder subkutan erfolgen, wobei eine intravenöse oder intraarterielle Verabreichung bevorzugt sind. Es ist ferner möglich die enterale, parenterale oder gentherapeutische Verabreichung des Inhibitors zu kombinieren. Für den Fall, dass der Inhibitor wiederholt über einen längeren Zeitraum verabreicht werden muss, wird vorzugsweise ein Inhibitor ausgewählt, der oral verabreicht werden kann.

In einer bevorzugten Ausführungsform handelt es sich bei den Patienten um solche, die der ersten speziellen Unterklasse angehören. Bei diesem Verfahren zur Vorbeugung oder Behandlung von Atherosklerose vor einem kardiovaskulären Primärereignis wird der Inhibitor vorzugsweise oral verabreicht.

In einer weiteren bevorzugten Ausführungsform handelt es sich bei den Patienten um solche, die der zweiten speziellen Unterklasse angehören. Bei diesem Verfahren zur Vorbeugung oder Behandlung von Atherosklerose vor einem kardiovaskulären Primärereignis wird der Inhibitor vorzugsweise oral verabreicht.

In einer weiteren bevorzugten Ausführungsform handelt es sich bei den Patienten um solche, die der ersten oder zweiten speziellen Unterklasse angehören, wobei der Inhibitor als Vorstufe über einen Vektor in die Endothelzellen der Gefäße verabreicht wird, um den Inhibitor in den Endothelzellen als Expressionsprodukt einer oder mehrerer Nukleotidseuenzen des Vektors bereitzustellen. Dieser Ansatz hat den Vorteil, dass eine wiederholte Verabreichung eines Medikamentes über einen längeren Zeitraum an den Patienten vermieden wird.

Im folgenden wird die Erfindung in weiteren Einzelheiten beschrieben.

Die Inhibierung von IKK-β kann auf der DNA-Ebene dadurch erfolgen, dass die Expression des Proteins verhindert wird. Eine Inhibierung kann auf allen Stufen der Genexpression ansetzen, so z. B. an dem Gen selbst oder an Expressionprodukten, wie RNAs und Proteinen. Aus dem Stand der Technik sind verschiedene Verfahren zur strukturellen Veränderung von Genen bekannt. Insbesondere sei auf die Verwendung von speziellen Vektoren verwiesen. In den Beispielen wird eine spezielle Verwendung eines Vektors beschrieben. Die Expression von IKK-β kann außerdem verhindert werden durch die Hemmung der Transkription oder der Translation des IKK-β -Gens bzw. der entsprechenden mRNA. Beispiele für solche Inhibitoren sind Antisense-RNAs und Ribozyme.

Die Inhibierung von IKK-β kann aber auch auf der Proteinebene dadurch erfolgen, dass die Funktion des exprimierten IKK-β-Proteins inhibiert wird. Eine Inhibierung auf der Proteinebene kann mit Proteinen oder Nicht-Proteinen erfolgen. Beispiele für Inhibitorproteine sind Antikörper, die mit Epitopen von IKK-β reagieren, oder Proteine, die mit einer aktiven Stelle von IKK-β wechselwirken und die Phosphorylierungsaktivität verhindern. Beispiele für Nicht-Proteine sind Wirkstoffe, die mit einer aktiven Stelle von IKK-β wechselwirken und die Phosphorylierungsaktivität verhindern. Für spezielle Beispiele von molekularen Inhibitoren von IKK-β wird auf die US-A 5,939,302 ausdrücklich Bezug genommen.

Vorzugsweise ist der Inhibitor für IKK-β selektiv.

In einer bevorzugten Ausführungsform der vorliegenden Erfindung ist der Inhibitor eine Verbindung, die die Expression des IKK-β codierenden Gens hemmt, z.B ein Ribozym oder eine Antisense-RNA. In einer besonders bevorzugte Ausführungsform ist der Inhibitor eine Antisense-RNA, die zu der von dem IKK-β codierenden Gen transkribierten mRNA oder einem Teil davon, vorzugsweise dem codierenden Bereich, komplementär ist und an diese mRNA spezifisch binden kann, wodurch die Synthese von IKK-β verringert oder gehemmt wird. In einer weiteren besonders bevorzugten Ausführungsform ist der Inhibitor ein Ribozym, das zu der von dem IKK-β codierenden Gen transkribierten mRNA oder einem Teil davon komplementär ist und an diese mRNA spezifisch binden and diese spalten kann, wodurch die Synthese von IKK-β verringert oder gehemmt wird. Geeignete Verfahren zur Herstellung von Antisense-RNA werden in EB-B1 0 223 399 oder EP-A1 0 458 beschrieben. Ribozyme bestehen aus einem einzelnen RNA-Strang und können andere RNAs intermolekular spalten, beispielsweise die von den IKK-β codierenden Sequenzen transkribierten mRNAs. Diese Ribozyme müssen prinzipiell über zwei Domänen verfügen, (1) eine katalytische Domäne and (2) eine Domäne, die zu der Ziel-RNA komplementär ist und an diese binden kann, was die Voraussetzung für eine Spaltung der Ziel-RNA ist. Ausgehend von in der Literatur beschriebenen Vorgehensweisen ist es inzwischen möglich, spezifische Ribozyme zu konstruieren, die eine gewünschte RNA an einer bestimmten, vorgewählten Stelle schneiden (siehe beispielsweise Tanner *et al*. , in: Antisense Research and Applications, CRC Press, Inc. (1993), 415-426).

In einer weiteren bevorzugten Ausführungsform ist der Inhibitor ein Vektor.

In einer weiteren bevorzugten Ausführungsform ist der Inhibitor ein Antikörper, der an IKK-β bindet, oder ein Fragment davon. Diese Antikörper können monoclonale, polyclonale oder synthetische Antikörper sein oder Fragmente davon. In diesem Zusammenhang bedeutet der Begriff "Fragment" alle Teile des monoclonalen Antikörpers (z.B. Fab-, Fv- oder "single chain Fv"-Fragmente), die die gleiche Epitopspezifität wie der vollständige Antikörper aufweisen. Vorzugsweise handelt es sich bei den erfindungsgemäßen Antikörpern um monoclonale Antikörper. Der monoclonale Antikörper kann ein aus einem Tier (z.B. Maus) stammender Antikörper, ein humanisierter Antikörper oder ein chimärer Antikörper oder ein Fragment davon sein. Chimäre, menschlichen Antikörper ähnelnde oder humanisierte Antikörper besitzen eine herabgesetze potentielle Antigenität, jedoch ist ihre Affinität gegenüber dem Ziel nicht herabgesetzt. Die Herstellung von chimären and humanisierten Antikörpern bzw. von den menschlichen Antikörpern ähnelnden Antikörpern wurde ausführlich beschrieben (siehe beispielsweise Queen et al., Proc. Natl. Acad. Sci. USA 86 (1989), 10029, and Verhoeyan et al., Science 239 (1988), 1534). Humanisierte Immunglobuline weisen variable Grundgerüstbereiche auf, die im wesentlichen von einem humanen Immunglobulin stammen (mit der Bezeichnung Akzeptor-Immunglobulin) und die Komplementarität der determinierenden Bereiche, die im wesentlichen von einem nicht-menschlichen Immunglobulin (z.B. von der Maus) stammen (mit den Bezeichnung Donor-Immunglobulin). Die (der) konstante(n) Bereich(e) stammt (stammen), falls vorhanden, auch im wesentlichen von einem menschlichen Immunglobulin. Bei der Verabreichung an menschliche Patienten bieten humanisierte (sowie die menschlichen) Antikörper eine Reihe von Vorteilen gegenüber Antiköpern von Mäusen oder anderen Spezies: (a) das menschliche Immunsystem sollte das Grundgerüt oder den konstanten Bereich des humanisierten Antiköpers nicht als fremd erkennen and daher sollte die Immunantwort gegen einen solchen injizierten Antiköper geringer ausfallen als gegen einen vollständig fremden Maus-Antikörper oder einen partiell fremden chimären Antikörper; (b) da der Effektorbereich des humanisierten Antikörpers menschlich ist, interagiert er besser mit anderen Teilen des menschlichen Immunsystems, und (c) injizierte, humanisierte Antikörper weisen eine Halbwertszeit auf, die im wesentlichen zu den von natürlich vorkommenden menschlichen Antikörpern äquivalent ist, was es erlaubt, kleinere and weniger häufige Dosen im Vergleich zu Antikörpern anderer Spezies zu verabreichen.

Ebenso können direkt an Proteine bindende Nukleinsäuren, sogenannte Aptamere, verwendet werden. Diese werden anhand der gereinigten Proteine aus großen Bibliotheken chemisch modifizierter RNAs identifiziert und gereinigt.

Die Aptamer-, Ribozym-, Anti-sense-RNA- und Antikörperinhibitoren können als solche oder aber vorzugsweise über eine Gentherapie verabreicht werden, wobei geeignete codierende DNA-Sequenzen vorzugsweise in einen Expressionsvektor inseriert und zu dem Zielgewebe gebracht werden. Somit umfaßt die vorliegende Erfindung auch Expressionsvektoren die diese Inhibitoren codierende DNA-Sequenzen enthalten. Die Bezeichnung "Vektor" bezieht sich auf einen Virus oder auf ein anderes geeignetes Vehikel. Die DNA-Sequenzen sind im Vektor mit regutatorischen Elementen funktionell verknüpft, die deren Expression in eukaryotischen Wirtszellen erlauben. Solche Vektoren enthalten neben den regulatorischen Elementen, beispielsweise einem Promotor, typischerweise einen Replikationsursprung und spezifische Gene, die die phänotypische Selektion einer transformierten Wirtszelle erlauben. Geeignete regulatorische Sequenzen sind ausserdem beschrieben in Goeddel: Gene Expression Technology: Methods in Enzymology 185, Academic Press, San Diego, CA (1990). Zu geeigneten Expressionsvektoren für Säugerzellen zählen beispielsweise von pMSXND, pKCR, pEFBOS, cDM8 und pCEV4 sowie von pcDNAI/amp, pcDNAZ/neo, pRc/CMV, pSV2gpt, pSV2neo, pSV2-dhfr, pTk2, pRSVneo, pMSG, pSVT7, pko-neo und von pHyg stammende Vektoren.

Vorzugsweise werden die vorstehend beschriebenen DNA-Sequenzen in einen für die Gentherapie geeigneten Vektor insertiert, beispielsweise unter Kontrolle eines gewebespezifschen Promotors, und in die Zellen eingeschleust. In einer bevorzugten Ausführungsform ist der die vorstehend beschriebenen DNA-Sequenzen enthaltende Vektor ein Virus, beispielsweise ein Adenovirus, ein adeno-assoziierter Virus, Vaccinia-Virus oder Retrovirus. Beispiele für geeignete Retroviren sind MoMuLV, HaMuSV, MuMTV, RSV oder GaLV. Besonders bevorzugt sind Adenoviren, insbesondere solche mit E1 und/oder E3-Mutationen (Deletionen), die zusätzlich auch eine E4-Mutation (Deletion) aufweisen können, oder sogenannte "gutless" Adenoviren. Für die Gentherapie geeignete Vektoren werden im übrigen in WO 93/04701, WO 92/22635, WO 92/20316, WO 92/19749 and WO 92/06180 offenbart. Für Zwecke der Gentherapie können die erfindungsgemäßen DNA-Sequenzen auch in Form von kolloidalen Dispersionen zu den Zielzellen transportiert werden. Dazu zählen beispielsweise Liposomen oder Lipoplexe (Mannino et al. , Biotechniques 6 (1988) , 682) .

Allgemeine, auf dem Fachgebiet bekannte Verfahren können zur Konstruktion von Expressionsvektoren, die diese DNA-Sequenzen und geeignete Kontrollsequenzen enthalten, verwendet werden. Zu diesen Verfahren zählen beispielsweise *in vitro* Rekombinationstechniken, synthetische Verfahren, sowie in vivo-Rekombinations-verfahren, wie sie in gängigen Lehrbüchem beschrieben sind.

Die vorstehenden Verbindungen bzw. die diese codierenden DNA-Sequenzen oder Vektoren werden gegebenenfalls mit einem pharmazeutisch verträglichen Träger verabreicht.

Geeignete Träger und die Formulierung derartiger Arzneimittel sind dem Fachmann bekannt. Zu geeigneten Trägern zählen beispielsweise Phosphat-gepufferte Kochsalzlösungen, Wasser, Emulsionen, beispielsweise Öl/Wasser-Emulsionen, Netzmittel, sterile Losungen etc Die geeignete Dosierung wird von dem behandelnden Arzt bestimmt und hängt von verschiedenen Faktoren ab, beispielsweise von dem Alter, dem Geschlecht, dem Gewicht des Patienten, der Art und dem Stadium der Herzerkrankung, oder der Art der Verabreichung.

In einer weiteren bevorzugten Ausführugsform ist der Inhibitor ein Wirkstoff, der ausgewählt wird aus der folgenden Gruppe:

Sulindac (vergl. Y. Yamamoto et al. J. Biol. Chem. 1999; 274 (38): 27307-14); NEMO-binding domain peptide (vergl. May Mj. et al. Science 2000; 289: 1550-4); 3-[(4-methylphenyl)-sulfonyl]-2-propennitril und 3-[(4-t-butylphenyl-sulfonyl]-2-propennitril, (vergl. J. W. Pierce et al. J. Biol. Chem. 1997; 272(34)).

Ferner stellt die vorliegende Erfindung ein Screeningverfahren zur Auffindung von selektiven Inhibitoren von IKK-β bereit, bei dem aktivierte Endothelzellen eingesetzt werden, um die Wirksamkeit von potentiellen Wirkstoffen speziell bei der Verhinderung einer Sekretion von MCP-1 zu testen.

Eine emiedrigte oder vollständig gehemmte Enzymaktivität bzw. Genexpression zeigt an, dass die Testverbindung als Inhibitor wirksam ist. Geeignete Assays sind dem Fachmann bekannt. Dieses Verfahren wird in einem zellulären Assay durchgeführt, bei dem Endothelzellen eingesetzt werden. Bei den Testverbindungen kann es sich um sehr unterschiedliche Verbindungen handeln, sowohl um natürlich vorkommende als auch synthetische, organische und anorganische Verbindungen, sowie um Polymere (z.B. Oligopeptide, Polypeptide, Oligonucleotide und Polynucleotide) sowie kleine Moleküle, Antikörper, Zucker, Fettsäuren, Nucleotide und Nucleotid-Analoge, Analoge von natürlich vorkommenden Strukturen (z.B. Peptid-"Imitatoren", Nucleinsäureanaloge etc.) und zahlreiche weitere Verbindungen. Darüber hinaus kann eine große Anzahl von möglicherweise nützlichen, IKK-β hemmenden Verbindungen in Extrakten von natürlichen Produkten als Ausgangsmaterial gescreent werden. Solche Extrakte können aus einer großen Anzahl von Quellen stammen, beispielsweise den Spezies Pilze, Actinomyceten, Algen, Insekten, Protozoen, Pflanzen und Bakterien. Die Extrakte, die Aktivität zeigen, können dann zur Isolierung des aktiven Moleküls analysiert werden. Siehe beispielsweise Turner, J. Ethnopharmacol. 51 (1-3) (1996), 3943 und Suh, Anticancer Res. 15 (1995) 233-239. Grundsätzlich geeignete Assay-Formate für die Identifizierurg von Testverbindungen, die die Expression oder Aktivität von IKK-β beeinflussen, sind in der biotechnologischen und pharmazeutischen Industrie gut bekannt und für den Fachmann sind zusätzliche Assays und Variationen dieser Assays offensichtlich. Änderungen im Expressionsspiegel IKK-β können unter Verwendung von dem Fachmann gut bekannten Verfahren untersucht werden. Dazu zählt die Überwachung der mRNA-Konzentration (z . B . unter Verwendung geeigneter Sonden oder Primer), Immunassays hinsichtlich der Proteinkonzentration RNAse-Schutzassays, Amplifikationsassays oder jedes undere für einen Nachweis geeignete Mittel, das auf dem Fachgebiet bekannt ist.

Das Suchen nach Inhibitoren kann auch in großem Maßstab erfolgen, beispielsweise dadurch, dass eine sehr hohe Anzahl von Kandidaten-Verbindungen in Substanzbibliotheken gescreent wird, wobei die Substanzbibliotheken synthetische und/oder natürliche Moleküle enthalten können. Jedenfalls können die Herstellung und das simultane Screenen von großen Banken aus synthetischen Molekülen mittels gut bekannter Verfahren der kombinatorischen Chemie ausgeführt werden, siehe beispielsweise van Breemen, Anal. Chem. 69 (1997), 2159-2164 und Lam, Anticancer Drug Des. 12 (1997) , 145-167 . Das erfindungsgemäße Verfahren kann auch als Screening mit hohem Durchsatz ("high throughput screening") stark beschleunigt werden. Die hier beschriebenen Assays können zur Verwendung in einem solchen Verfahren entsprechend modifiziert werden.

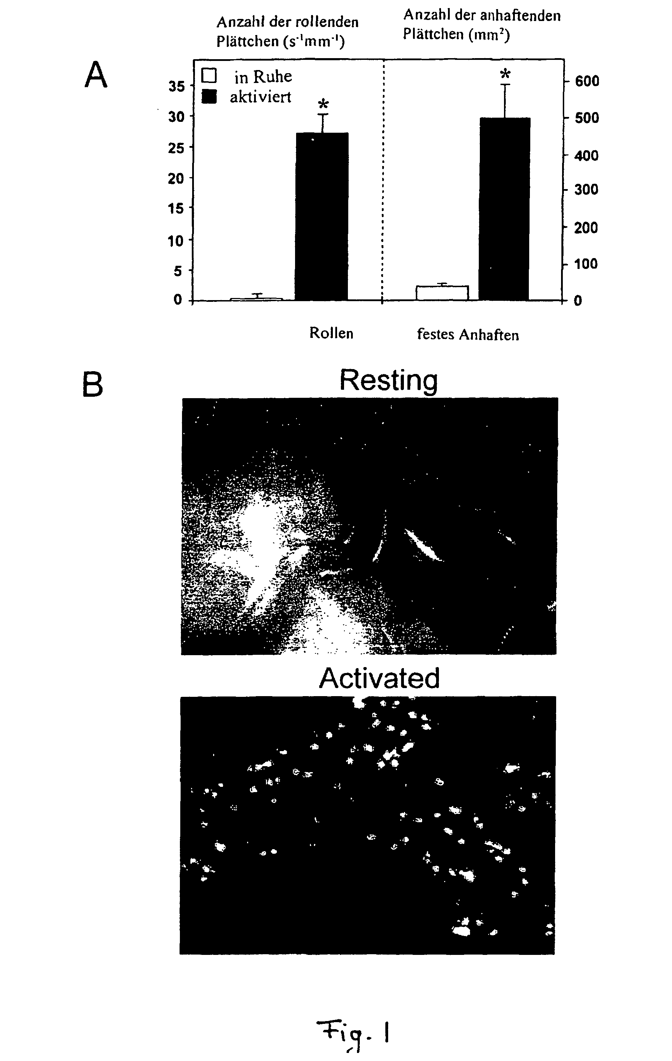

Figur 1 zeigt Blutplättchen-Endothelzellwechselwirkungen *in vivo.* Die Blutplättchen-Endothelzellwechselwirkungen wurden mit Hilfe von intravitaler Mikroskopie in Arteriolen der Mikrovaskulatur eines Darms von Mäusen, die ischämischen Bedingungen unterworfen wurden (AKTIVIERT, activated), untersucht. Sham-Tiere dienten als Kontrollen (in Ruhe, resting). Fig. 1A: Blutplättchen wurden gemäß ihrer Wechselwirkungen mit Endothelzellen, wie im Stand der Technik beschrieben (1, 2), klassifiziert: Rollende Blutplättchen werden angegeben als Anzahl der Zellen pro Sekunde und mm Gefäßdurchmesser (Rolling); anhaftende Blutplättchen werden angegeben als Anzahl pro mm² Gefäßoberfläche (Firm adhesion). Mittelwert +- SEM, n=6 Versuchstiere pro Gruppe. *p<0,01 gegenüber sham (Dunn'sche Methode). Fig 1B: Rhodamin-6G-markierte Plättchen wurden in Arteriolen unter Verwendung von intravitaler Fluoreszenzmikroskopie sichtbar gemacht. Obwohl nur wenige Blutplättchen am Endothel in sham-Tieren anhaften, ist ein Anhaften von Blutplättchen an Endothelzellen unter Ischämiebedingungen signifikant erhöht. Vergrößerung der Aufnahmen: x450.

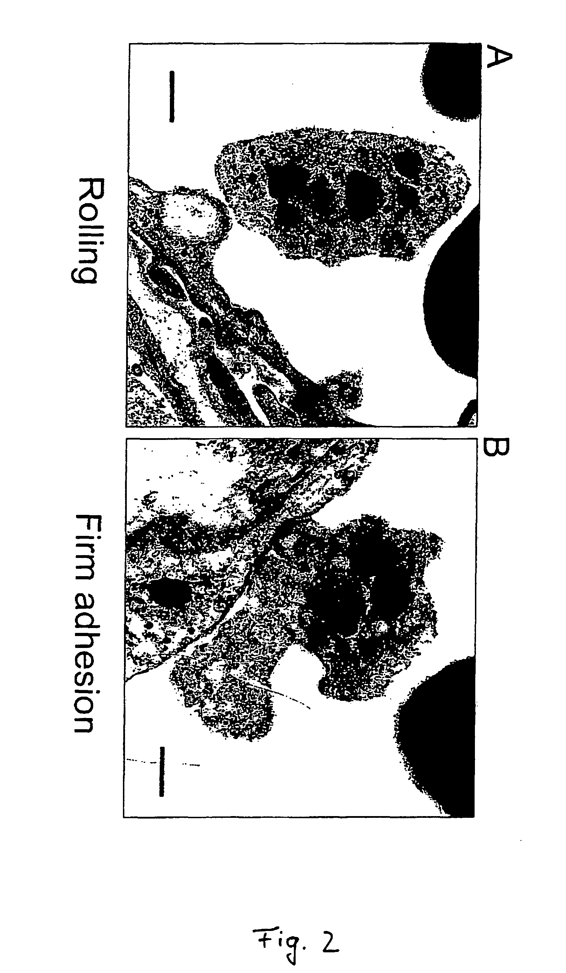

Figur 2 zeigt eine durch Elektronenmikroskopie sichtbar gemachte Blutplättchen-Endothel-Adhäsion auf einer endothelialen Monoschicht. Elektronenmikroskopie wurde eingesetzt, um zu zeigen, dass Blutplättchen an eine intakte postischämische endotheliale Monoschicht anhaften. Fig 2A: Ein rollendes Blutplättchen wird mit losem Kontakt zum Endothel gezeigt. Man beachte das normale Aussehen und die normale Verteilung der Blutplättchengranula. Figur 2B zeigt ein Blutplättchen, das fest an der endothelialen Monoschicht anhaftet. In Bereichen der Aufspreitung und des festen Anhaftens ist das Blutplättchen eine signifikante Degranulation eingegangen, was auf eine Ausscheidung von aus dem Plättchen stammenden Verbindungen hinweist. Vergrößerung: x20,000. Balken bedeuten 0,5µm.

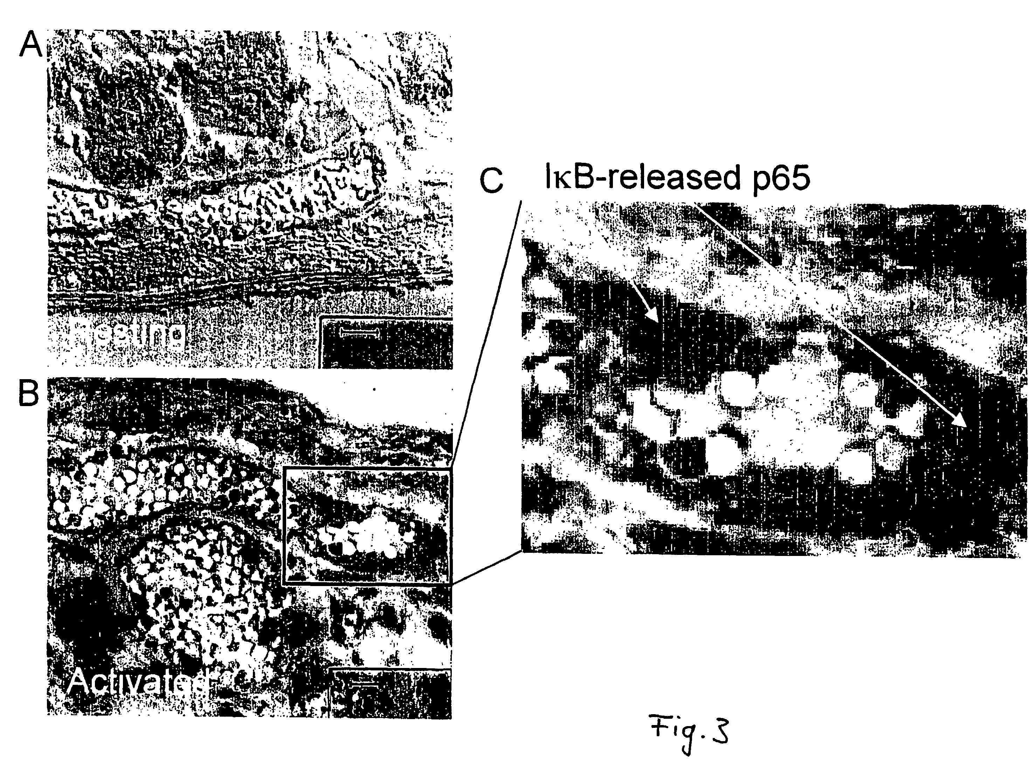

Figur 3 zeigt, dass eine Adhäsion von Blutplättchen an das Endothel in Zusammenhang steht mit einer Aktivierung von NF-κB. Kryostatschnitte von Eingeweideproben wurden mit einem monoklonalen Antikörper (mAb) gegen aus IκB abgegebenem p65 (=aktiviertem NF-κB) inkubiert, gefolgt von einem Anfärben mit Hilfe der Peroxidase-Antiperoxidase-Technik. Eine p65 Immunreaktvität wird durch das rote (in der Figur dunkel erscheinende) Reaktionsprodukt angezeigt. Fig. 3A: Bei Kontrolltieren (Resting) war im Gefäßendothel eine Immunreaktivität von p65 so gut wie nicht vorhanden. Figur 3B: In Experimenten mit verstärkter Blutplättchen-Endothel-Adhäsion (Activated) war eine p65 Anfärbung in Arteriolen und dem umgebenden Gewebe verstärkt zu finden. Figur 3C: In Bereichen mit massiver Blutplättchenakkumulation, die in einer Vergrößerung eines Ausschnitts von Figur 3B gezeigt wird, lag durch IκB abgegebenes p65 vorwiegend in der endothelialen Monoschicht vor (Pfeile). Vergößerung: x40 bei Figur 3A und 3B sowie x100 in Figur 3C. Balken bedeuten 10µm.

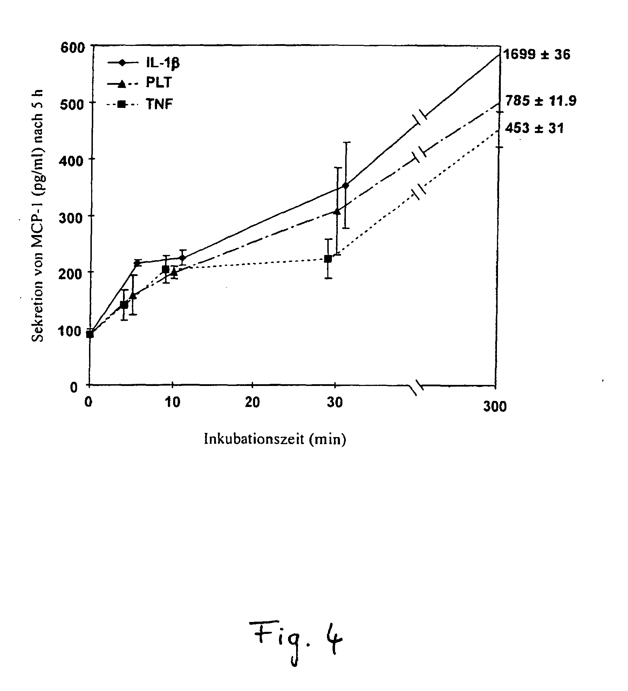

Figur 4 zeigt den Effekt einer vorübergehenden Wechselwirkung von aktivierten Blutplättchen mit HUVEC auf die Sekretion von MCP-1. HUVEC wurden entweder mit oder ohne α-Thrombin-aktivierten Blutplättchen (PLT; 2x10⁸/ml) IL-1β (100pg/ml) oder TNF (1,67ng/ml) für 5 bis 300 min behandelt. Anschließend wurden die Blutplättchen bzw. Cytokine durch Waschen entfernt und es wurde neues Kulturmedium zugesetzt. Die Zellen wurden insgesamt für 5 Stunden inkubiert. Anschließend wurde der Überstand abgesaugt und in einem ELISA zur Messung von MCP-1 eingesetzt. Die Kurven zeigen den Mittelwert +- Standardabweichung aus vier unabhängigen Experimenten, die jeweils dreifach durchgeführt wurden.

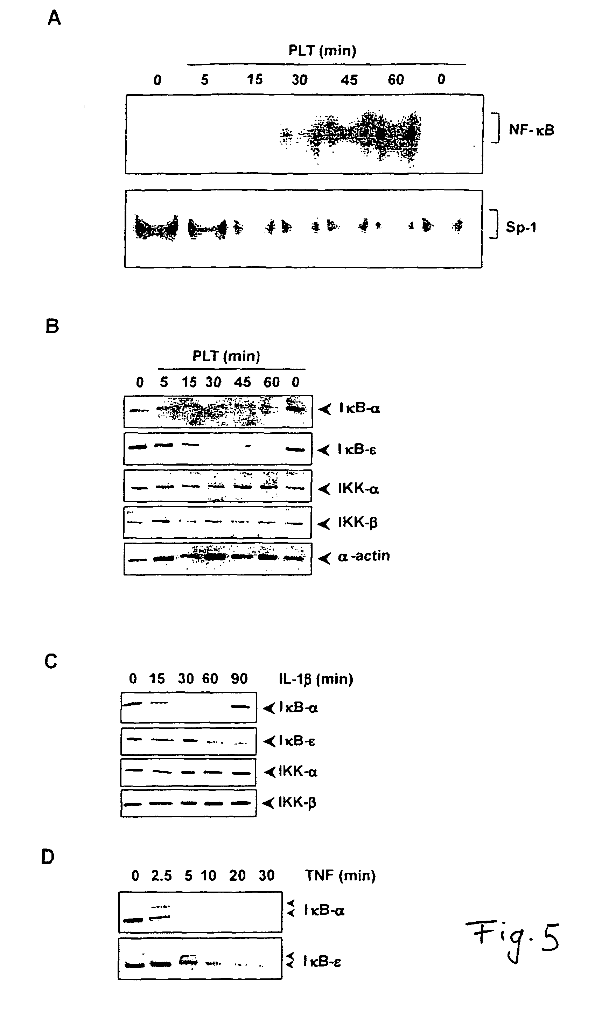

Figur 5 zeigt die Stimulierungs-abhängige NF-κB Aktivierung und den IκB Abbau in HUVEC. Figur 5A: Kernextrakte aus mit Blutplättchen stimulierten HUVEC (PLT; 2x10⁸/ml) wurden mit EMSA auf NF-κB oder Sp-1 Bindung untersucht, was in Klammern gezeigt wird. Figur 5B: Cytosolextrakte aus mit Blutplättchen stimulierten Zellen wurden mit Hilfe von Western-Blots analysiert. Die Proteine IκB-α, IκB-ε, IKK-α und IKK-β werden durch die Pfeilspitzen gezeigt. α-Actin diente als Loading-Kontrolle. Figur 5C: Zytosolextrakte aus mit IL-β stimulierten Zellen (100 pg/ml) wurden mit Hilfe von Western-Blots untersucht. Figur 5D: Zellen wurden mit TNF inkubiert(1,67 ng/ml) und Zytosolextrakte wurden mit Hilfe von Western-Blots untersucht. Der obere Pfeil zeigt die phosphorylierten Formen von IκB-α oder -ε an.Ein repräsentativer Blot wird für alle Fälle gezeigt (n=3).

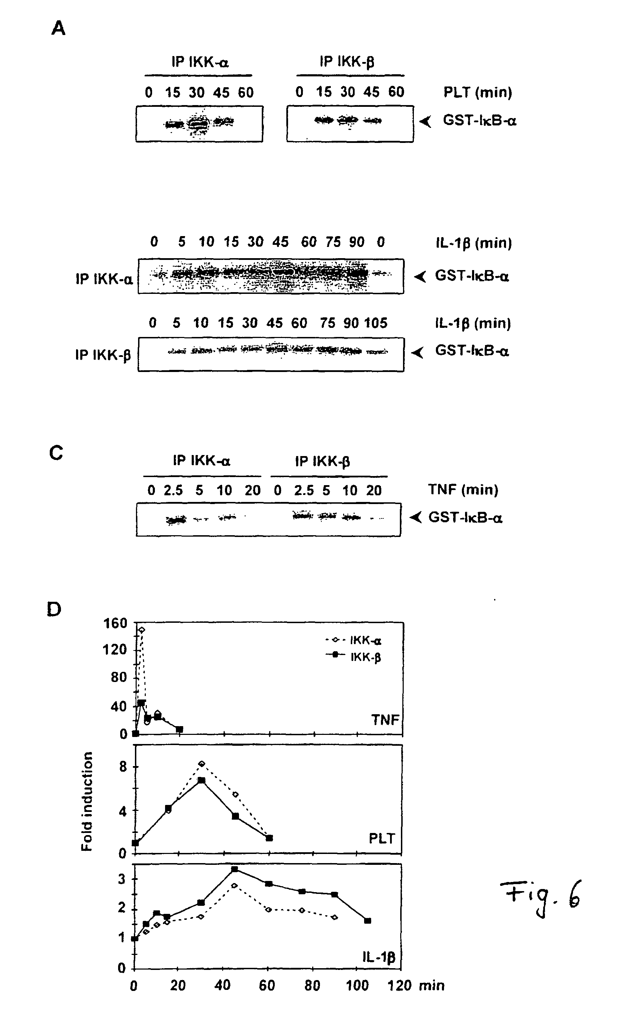

Figur 6 zeigt die differentielle IKK-Kompfexaktivierung in Endothelzellen. Zellen wurden wie angegeben inkubiert und Zytosolextrakte wurden hergestellt. Einer Immunopräzipitation (IP) mit Antikinase-Antikörpern wie angegeben folgte ein Kinase-Assay unter Verwendung des Substrats GST.IκB-α in Gegenwart von ³²P-ATP. Eine zeitabhängige Phosphorylierung des Substrats wird gezeigt jeweils nach Stimulierung von HUVEC mit Blutplättchen (PLT; 2x10⁸/ml)(Figur 6A), IL-1β (100pg/ml)(Figur 6B) oder TNF (1,67ng/ml) (Figur 6C). Ein repräsentatives Experiment wird für jeden Fall gezeigt (n=3). Figur 6D: die Kinase-Assays wurden gescanned und densiometrisch analysiert. Das Diagramm zeigt die Faktoren der Induktion der IKK-Aktiviät gegenüber der Kontrolle nach einer Stimulierung.

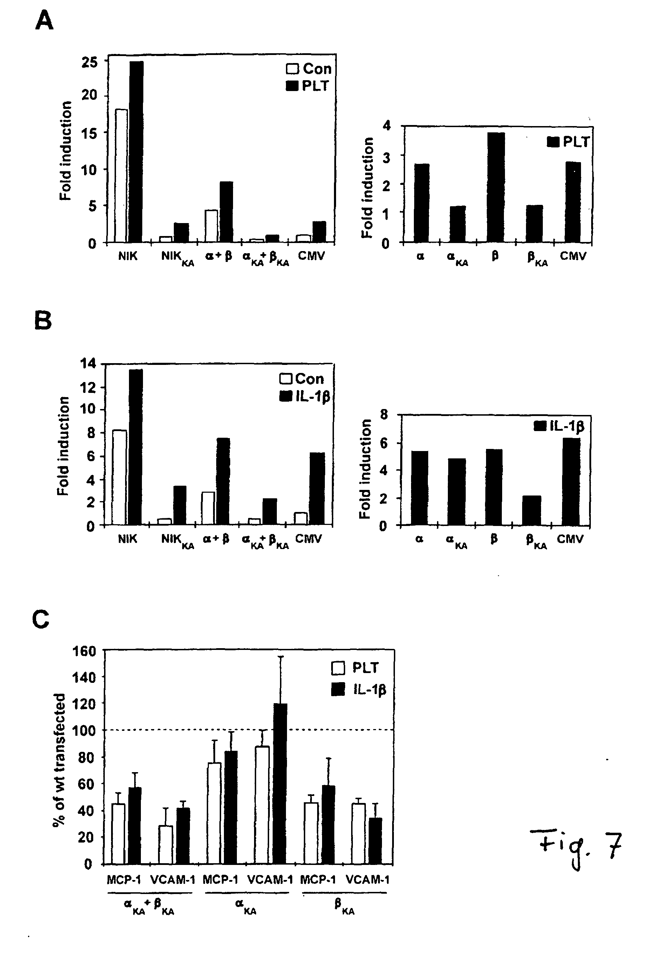

Figur 7 zeigt, dass die κB- und MCP-1/VCAM-1 Promotor-abhängige Transkription inhibiert wird durch Überexpression von dominant-negativen IKK-Komplexkomponenten Figur 7A und Figur 7B: ECV304 Zellen wurden cotransformiert mit 3kB.luci, pRLtk und Kinase-Überexpressionsplasmiden (IKK-α, -β, oder NIK, Wildtyp oder mutiert, oder einem leeren Vektor, CVM) in Einzel- oder Doppeltransformationen, wie angegeben. Nach einer Inkubation über Nacht wurden die Zellen stimuliert mit entweder aktivierten Blutplättchen (PLT; 108/ml) (Figur 7A), oder IL-1β (100pg/ml) (Figur 7B) für 60 Minuten. Nach weiteren 15 Stunden wurden die Zellen lysiert und der Glühwürmchen- und *Renilla*-RLU wurde gemessen. Die Ergebnisse werden gezeigt als Vielfachen der Induktion gegenüber derjenigen, die mit unstimulierten CMV-transformierten Zellen erhalten wurden. Die linken Balken zeigen den Effekt von entweder Wildtyp (wt) oder mutiertem NIK sowie von IKK-α und - β auf die Stimulus-induzierte κB-abhängige Transkription. Repräsentative Versuche werden gezeigt (n = 3). Con; unstimulierte Zellen. Fig. 7C: Zellen wurden wie angegeben entweder mit einem MCP-1- oder VCAM-1- promotorabhängigen Luciferase-Reporterplasmid zusammen mit pRLtk und IKK über Expressionsplasmide transformiert. Nach einer Stimulierung mit Blutplättchen oder IL-1β wurde Glühwürmchen- und *Renilla*-RLU gemessen. Die Ergebnisse werden gezeigt als Prozentsatz der Inhibierung, die relativ zu dem mit dem Wildtyp (wt) transformierten Zellen erhalten wurden, die mit der gepunkteten Linie bei 100% angegeben werden. Die Balken zeigen den Mittelwert ±Standardabweichung aus drei unabhängigen Experimenten. α, IKK-α; β, IKK-β: KA, die Kinase-aktive Stelle ist mutiert.

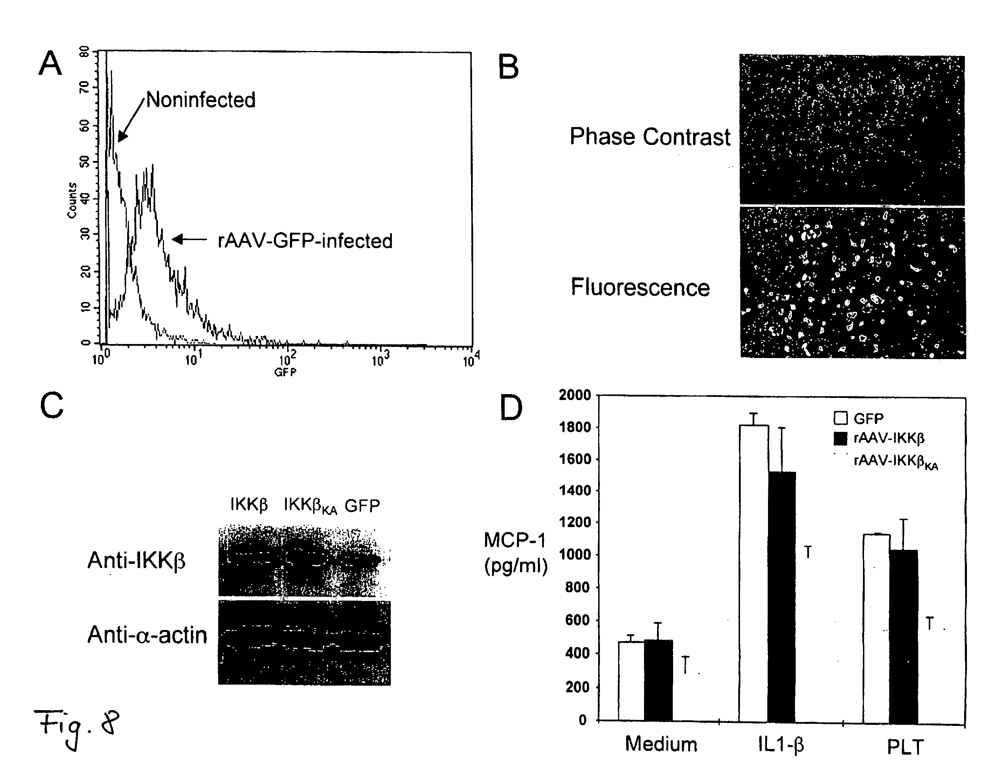

Figur 8 zeigt, dass der rAAV-vermittelte dominant-negative IKK-β-Transfer eine Blutplättchenund IL-1β-induzierte endotheliale MCP-1-Sekretion inhibiert. Subkonfluente HUVEC wurden unbehandelt gelassen, oder wurden mit Kontroll- rAAV-GFP, Wildtyp rAAV-IKK-β oder dominant negativen Mutanten rAAV-IKK-β_{KA} transformiert. 48 Stunden nach einer Transformation wurden die Zellen unbehandelt gelassen oder mit aktivierten Blutplättchen (PLT; 2x10⁸/ml) oder IL-1β (100 pg/ml) für 60 Minuten stimuliert, gewaschen und weiter wie angegeben inkubiert. Fig. 8A: die Zellen wurden auf GFP Expression mit Hilfe von Durchflusscytometrie untersucht, um eine Transformationseffizienz zu bestimmen. Die mit non-infected gekennzeichnete Kurve zeigt nicht infizierte Zellen und die mit rAAV-GFP-infected markierte Kurve zeigt Zellen an, die mit rAAV-GFP behandelt wurden. Etwa 50% der HUVEC werden mit rAAV-GFP infiziert. Fig. 8B: eine GFP Expression wurde durch Fluoreszenzmikroskopie analysiert. Oberes Bild: Phasenkontrast der HUVEC Monoschicht; unteres Bild: entsprechende Grün-Fluoreszenz (helle Punkte) Mikrographie. Fig. 8C: Cytosolextrakte von mit rAAV-IKK-β, rAAV-IKK-β_{KA} oder rAAV-GFP transformierte HUVEC wurden mit Hilfe von Western-Blots auf endogenes (GFP) und überexprimiertes IKK-β-Protein untersucht. α-Actin wurde als Loading-Control verwendet. Fig. 8D: 4 Stunden nach einem Waschen wurde lösliches MCP-1 im Überstand der Zellen mit Hilfe von ELISA quantitativ bestimmt. Die Daten werden als Mittelwert ±Standardabweichung aus jeweils drei doppelt durchgeführten Experimenten gezeigt.

Die Erfindung wird nachfolgend anhand spezieller Beispiele weiter erläutert.

### BEISPIELE

### Materialien und Methoden

### Tiere und intravitale Fluoreszenzmikroskopie

Weibliche Balb/c Mäuse (Charles River, Sulzfeld) im Alter von 5 bis 7 Wochen (10 Versuchstiere, 10 Plättchenspender) wurden verwendet. Die chirurgische Prozedur ist Stand der Technik (1, 2). Speziell wurde ein Segment des Jejenums nach außen gelegt und einer 60 minütigen normothermischen Ischämie unterworfen. Blutplättchen der Mäuse wurden aus Vollblut isoliert und mit Rhodamin-6G markiert. Blutplättchen-Endothelzellwechselwirkungen in der postischämischen Mikrovaskulatur wurden in submukösen Arteriolen durch intravitale Mikroskopie vor und 60 Minuten nach der Ischämie untersucht. Fünf interessierende, nicht überlappende Bereiche wurden zufällig ausgewählt und eine quantitative Bestimmung von Plättchen-Endothelzellenwechselwirkungen wurde durchgeführt. Diese Wechselwirkungen wurden als rollende ("Rolling") oder feste Adhäsion von Plättchen klassifiziert.

### Elektronenmikroskopie und Immunohistochemische Untersuchungen

Proben aus den Darmsegmenten wurden vor sowie 60 Minuten nach der Ischämie genommen. Für Zwecke der Elektronenmikroskopie wurde das Gewebe fixiert und, wie im Stand der Technik beschrieben, verarbeitet (2). Ultradünne Bereiche wurden geschnitten und mit Uranylacetat und Bleicitrat angefärbt und unter einem Zeiss EM 900 Transmissionselektronenmikroskop (Zeiss, Oberkochen), das bei 80 kV arbeitet, untersucht. Für Zwecke des Immunfärbens wurden die Biopsien in eine O.C.T. Verbindung (Tissue-Tek; Miles Inc., Elkhart, IN, USA) gegeben und sofort mit flüssigem Stickstoff gefroren. Um aktiviertes NF-κB sichtbar zu machen, wurden Aceton-fixierte Cryostatsschnitte (6 µm) mit Biotin-konjugierten Maus-Anti-Mensch α-p65mAb (Roche Diagnostics, Mannheim) inkubiert, der selektiv mit p65 nur in Abwesenheit von IκB reagiert und daher die aktivierte Form von NF-κB erkennt und der mit dem entsprechenden Mäuseantigen über Kreuz reagiert (3). Nach einer Inkubation mit dem primären Antikörper wurden die Sektionen mit Peroxidase lmmunohistochemie- Kits angefärbt (Vectastain, Camon, Wiesbaden). Eine endogene Peroxidaseaktivität wurde mit Methanol-H₂O₂ für 10 Minuten bei Raumtemperatur blockiert.

### Zellkultur

Primäre Kulturen aus menschlichen Nabelvenen-Endothelzellen (HUVEC) wurden durch Collagenase-Behandlung von Nabelvenen wie in (4) beschrieben erhalten. Zellen aus 4 - 6 Präparationen wurden vereinigt und auf Kulturplatten mit 24 Vertiefungen in komplettem Endotheizellwachstumsmedium (PromoCell, Heidelberg) kultiviert, das 2% FCS, 1µg/ml Hydrocortison, 0,1 ng/ml hEGF, 1,0 ng/ml FGF, 50 µg/ml Gentamycin und 2,5 µg/ml Amphotericin B enthielt.

Die menschliche Endothelzell-Linie ECV304 (American Tissue Culture Collection, Rcokville, MD, USA) wurde in M199, das mit 10% FCS, 1% Natriumpyruvat, 1 mM L-Glutamin, 100 U/ml Penicillin, 100 µg/ml Streptomycin (Biochrom, Berlin) angereichert war, kultiviert. Um eine Endotoxinkontamination zu eliminieren, wurden alle Kristalloid-Lösungen ultrafiltriert (U 200, Gambro, Hechingen), und Mutterlösungen von Proteinen wurden mit Polymyxin-Säulen (Pierce, Rockford, IL, USA) dekontaminiert. Um eine Endotoxin-Kontamination auszuschließen, wurden alle Zellsuspensionen durch einen farbstoffbildenden Limulus Amoebocyt-Lysatassay (Schutz, Heidelberg,) nach jedem Experiment evaluiert.

### Bestimmung von MCP-1

Die Konzentrationen von MCP-1-Protein wurden in HUVEC-Überständen durch ELISA (Quantikine R&D, Wiesbaden-Nordenstadt) bestimmt.

### Co-Kultivierung von Endothel-Monoschichten mit Blutplättchen

Blutplättchen wurden aus Säure-Citrat-Dextrose-anticoaguliertem Vollblut isoliert, das aus gesunden Personen, die Nichtraucher waren und die unter keinem Medikamenteneinfluß standen, von dem bekannt ist, dass er eine Blutplättchenfunktion beeinflusst, gesammelt. Die Blutplättchen wurden gewaschen und in Tyrode'scher Lösung/HEPES-Puffer resuspendiert (HEPES 2,5 mM, NaCl 150 mM, NaHCO₃ 12 mM, KCl 2,5 mM, MgCl₂ 1mM, CaCl₂ 2 mM, D-Glycose 5,5 mM, 1 mg/ml BSA, pH 7,4), um eine Blutplättchenzahl von 4 x 10⁸/ml, wie in (5) beschrieben, zu schaffen. Blutplättchen wurden mit 2 U/ml α-Thrombin (Sigma, Deisenhofen) für 20 Minuten vorstimuliert, gefolgt von einer Thrombin-Antagonisierung mit 5 U/ml Hirudin (Roche Diagnostics). Eine Blutplättchenaktivierung wurde durch Bestimmung der P-Selectin Oberflächen-Expression und des Rezeptors von aktiviertem Fibrinogen (LIBS-1, Dr. Mark Ginsberg, Scripps Research Institute, La Jolla, CA, USA), verifiziert. 1 ml der Blutplättchensuspension wurde zu 1 ml komplettem Medium (Endzahl der Blutplättchen 2 x 10⁸/ml) zugesetzt und in Vertiefungen mit konfluenten HUVEC Monoschichten auf Platten mit 6 Vertiefungen, überführt. Die Zellen wurden unbehandelt gelassen oder mit Blutplättchen, wie angegeben, bei 37°C inkubiert.

Abschließend wurden die Blutplättchen durch mehrere vorsichtige Waschstufen entfernt, und Kulturmedium wurde zu den Vertiefungen zugesetzt bis die vollständige Inkubationszeit abgelaufen war. In vorläufigen Untersuchungen unter Verwendung eines Blutplättchenspezifischen Anti-CD42b mAb, wurde mikroskopisch und flusszytometrisch sichergestellt, dass durch das Waschen alle Blutplättchen von der Endothel-Monoschicht entfernt wurden.

### Electrophoretic mobility shift assay (EMSA=Gelretardierungsassay)

Kernextrakte der Zellen wurden wie im Stand der Technik beschrieben (6) hergestellt und analysiert. Das prototypische Immunoglobolin-κ-Ketten Oligonucleotid wurde als Sonde verwendet und mit dem Klenow-Fragment von DNA Polymerase I (Roche Diagnostics) markiert. Kernproteine wurden mit durch [α-³²P]dCTP (NEN Life Science Products, Brüssel, Belgien) radiomarkierte Sonden für 30 Minuten bei Raumtemperatur in Bindungspuffer (6) inkubiert. Proben wurden in 0,25x TBE auf nicht denaturierenden vierprozentigen Polyacrylamidgelen laufen gelassen. Die Bindung des Transkriptionsfaktors Sp-1 wurde unter Verwendung eines Konsensus-Oligonukleotids (Promega, Heidelberg), das mit [γ-³²P]ATP (NEN Life Science Products) und T4 Polynukleotid-Kinase (Roche Diagnostics) markiert war, analysiert. Die Gele wurden getrocknet und autoradiografisch analysiert.

### PAGE und Westem Blot Analyse

Zytosolextrakte wurden isoliert, und eine Elektrophorese und Blotting wurden wie im Stand der Technik beschrieben durchgeführt (6, 7). Nach Übertragung wurden die Membranen mit Antikörpern gegen IκB-α (Santa Cruz Biotechnology, Heidelberg), IκB-ε (Prof. N. Rice, NIH, Frederick, MD), IKK-α, IKK-β (Santa-Cruz Biotechnology), oder α-Actin (Sigma) inkubiert. Die Proteine wurden auf Röntgenfilm unter Verwendung eines Western-Blot-Chemielumineszenzreagenzes (NEN Life Science Products), sichtbar gemacht. Proteingrößen wurden über Molekulargewichtsstandards (Amersham Pharmacia Biotech, Braunschweig, Bio-Rad, München) bestätigt.

### Immunopräzipitation und Kinase-Assay

Cytosolextrakte wurden einer Immunopräzipitation (IP) (7) in TNT Puffer unterworfen, der 1 mM Dithiothreitol, 0,5 µM 4-(2-Aminoethyl)-benzolsulfonylfluorid (AEBSF), und 0,75 µg/ml jeweils von Leupeptin, Antipain, Aprotinin, Pepstatin A, Chymostatin (Sigma) enthielt. Unspezifische Bindungen wurden durch Inkubation mit 1µg normalem Hasen-IgG (Santa Cruz Biotechnology) und 25 µl 6% Protein A-Agarose (Roche-Diagnostics) für 30 Minuten bei 4°C blockiert, gefolgt von einer Immunopräzipitation für 2 Stunden bei 4°C mit 1 µg geeignetem Anti-Kinase Antikörper (Santa Cruz Biotechnology). Nach dreimaligem Waschen jeweils mit TNT und Kinase-Puffer (20 mM HEPES, pH 8,0; 10 mM MgCl₂, 100 µM Na₃VO₄; 20 mM β-Glycerophosphat; 50 mM NaCl; 2 mM Dithiotreitol; 0,5 µM AEBSF; 0,75 µg/ml von jeweils Leupeptin, Antipain, Aprotinin, Pepstatin A, Chymostatin), wurden die ausgefällten Proteine durch einen Kinase-Assay analysiert. Die Reaktion wurde in einem Kinasepuffer für 30 Minuten bei 30°C in Gegenwart von 5 µCi [γ-³²P]ATP (NEN Life Science Products) und GST-IκB-α (Santa Cruz Biotechnology) durchgeführt. Proteine wurden mit PAGE analysiert und über Autoradiografie sichtbar gemacht.

### Transfektion von Endothelzellen

Die folgenden Reporterplasmide wurden bei Transfektionsuntersuchungen eingesetzt:
- 3xκB-luci, ein Luciferasereporterplasmid des Glühwürmchens, das 3 Kopien einer prototypischen κB-Stelle enthält (7);
- VCAM-1.luci, umfassend 1811 Basenpaare des VCAM-1-Promoterbereichs (Dr. Nigel Mackmann, Scripps Research Institute, La Jolla, CA); und
- MCT-1.luci, enthalten die proximalen Promoter- und distalen Enhancerbereiche des menschlichen MCP-1 Gens (8, 9) (Dr. Teizo Yoshimura, NIH, Frederick, MD).

Die folgenden Überexpressionsplasmide wurden eingesetzt:
- IKK-α,
- IKK-β, und
- NIK.

(Wildtyp und mutiert, Kinase-inaktive Formen, Dr. Mike Rothe, Tularik Inc., South San Francisco, CA).

Leeres RcCMV (Invitrogen, Groningen, Holland) wurde als Negativkontrolle eingesetzt. Diese Plasmide wurden, entweder allein oder in Kombination, transient mit einem konstitutiv aktiven *Renilla*-Luciferase-Kontrollplasmid, PRLtk (Promega), in Endothelzellen wie im Stand der Technik (10) beschrieben cotransformiert. Nach 24 Stunden wurden die Zellen für 60 Minuten mit entweder IL-1β oder Blutplättchen stimuliert, gefolgt von einer Kultivierung für weitere 15 Stunden. Nach der Stimulierung wurden die Zellen lysiert, und die Luciferase-aktivität wurde unter Verwendung des dualen Luciferase-Reporter-Assay-Systems (Promega) bestimmt. Die Ergebnisse werden als Verhältnis der relativen Lichteinheiten (RLU) von Feuerfliegen und Renilla-Luciferase ausgedrückt.

### Konstruktion und Reinigung eines rekombinanten Adeno-assoziierten Virus

Das Plasmid pTR-UF5 wurde von Dr. Muzyczka (Gene Therapy Center, Gainsville, Florida) (11, 12) und das Plasmid pDG wurde von Dr. Kleinschmidt (Deutsches Krebsforschungszentrum, Heidelberg, Deutschland) (11-13) bereitgestellt. Ein rekombinanter, replikationsdefizienter Adeno-assoziierter Virus (rAAV), der das grünfluoreszierende Protein (rAAV-GFP), den menschlichen Wildtyp IKK-β (rAAV-IKK-β) oder mutiertes IKK-β_{KA} (rAAV-IKK-β_{KA}) enthielt, wurden wie folgt konstruiert: Die Kodierungssequenz des Wildtyps oder des mutierten IKK-β wurden in die Polylinkersequenz des pTR-UF5 Vektors inseriert. Die erhaltenen Plasmide wurden anschließend mit dem pDG-Plasmid in subkonfluente HEK 293 Zellen und unter Verwendung einer modifizierten Kalziumphosphatcopräzipitationsmethode cotransformiert, und eine Erzeugung von rAAV wurde, wie im Stand der Technik (13, 14) beschrieben, durchgeführt. Die Titer von infektiösen viralen Partikeln (IVP) wurden durch Infektion von proliferierenden HeLa-Zellen beurteilt.

### Transduktion von Endothelzellen

HUVEC wurden auf Platten mit 96 Vertiefungen, bei 10⁴ Zellen pro Vertiefung, in 200 µl Kulturmedium ausplattiert . Bei Subkonfluenz, etwa 16 bis 24 Stunden später, wurden 1 bis 3 x 10³ IVP von rAAV zu jeder Zelle zugegeben. Die Medien wurden 16 Stunden nach Zugabe von rAAV gewechselt, und transduzierte Zellen wurden für weitere 48 Stunden kultiviert. Anschließend wurden die Zellen mit IL-1β oder α-Thrombin-aktivierten Blutplättchen für 60 Minuten stimuliert, und eine Sekretion von MCP-1 wurde 4 Stunden später evaluiert. Eine Effizienz von endothelialer rAAV-GFP Transduktion wurde durch Durchflusszytometrie, Fluoreszenzmikroskopie und Western Blot evaluiert.

### Ergebnisse

### Blutplättchen-Wechselwirkung in vivo

Das Mausmodell der Ischämie, das hier eingesetzt wurde, erlaubt die Unterscheidung verschiedener Arten der Blutplättchenadhäsion, die mit intravitaler Mikroskopie sichtbar gemacht wurde.

Im physiologischen Zustand *in vivo* wechselwirkten zirkulierende Blutplättchen selten mit dem mikrovaskulären Endothel (Figur 1A und B, ruhend). Wenige Blutplattchen wurden beobachtet, die entlang der Endothelzellauskleidung von Arteriolen entlangrollten (< 1/s/ml). Gleichzeitig wurden pro mm² Endothelzelloberfläche lediglich 38 ± 16 Blutplättchen gefunden, die fest anhafteten.

Im Gegensatz dazu wurde für den Fall, dass ähnliche Messungen in Arteriolen unter Ischämie-Bedingungen (60 min Ischemie) durchgeführt wurden, eine dramatisch erhöhte Blutplättchen-Endothelzelienadhäsion gefunden (Figur 1 A und B, aktiviert). Es wurde eine Erhöhung um das etwa 160-fache bei der Wechselwirkung unter Rollen und eine Erhöhung um das 13-fache der festen Plättchenadhäsion an ischämisches Endothel gefunden (P<0,01).

### Feste Adhäsion von Blutplättchen an das Endothel induziert eine Granulasekretion

Um morphologische Veränderungen zu untersuchen, die in Blutplättchen stattfinden, wenn diese an Gefäßendothel *in vivo* anhaften, wurden elektronenmikroskopische Untersuchungen durchgeführt. Es wurde gefunden, dass einzelne Blutplättchen auch in Abwesenheit von Aggregaten an Endothelzellen anhaften, ohne dass Defekte in der Endothelzellschicht erkennbar wären (Fig. 2). Diese lose an Endothelzellen anhaftenden Blutplättchen (Rolling) zeigten das typische Muster vollständiger Granula, die noch nicht degranuliert sind (Fig. 2a). Im Gegensatz dazu sind Blutplättchen, die über die Endothelzelloberfläche (feste Adhäsion) ausgebreitet sind, gekennzeichnet durch eine starke Sekretion ihrer Granula, wie durch leere Organellen, die in diesen Bereichen gefunden werden, belegt ist (Fig. 2B).

### NF-κB wird in der Mikrovaskulatur in Bereichen der Blutplättchenadhäsion aktiviert

Es ist aus *in vitro* Untersuchungen bekannt, dass eine Wechselwirkung aktivierter Blutplättchen mit Endothelzellen (permanente Co-Kultur) zu einer Genexpression von Chemokinen und Adhäsionsmolekülen über eine Aktivierung von NF-κB führt. Um die Aktivierung dieses Transkriptionsfaktors in Bereichen einer verstärkten Blutplättchen-Endotheladhäsion in der Mikrovaskulatur zu untersuchen, wurde eine Immunohistochemie durchgeführt, auf Cryostatschnitten unter Verwendung eines mAb gegen die von IκBfreigesetzte NF-κB p 65 Untereinheit. In sham-Mäusen, fehlt eine Aktivierung von NF-κB fast vollständig (Fig. 3A, Resting). Unter Bedingungen einer verstärkten Blutplättchen-Endotheiwechselwirkung wurde eine erhöhte Immunoreaktivität von aktiviertem NF-κB, gezeigt durch p65-Markierung, in der Mikrovaskulatur beobachtet (Fig. 3B, aktiviert) und vorwiegend in der Endothelmonoschicht benachbart zu den Blutplättchenaggregaten (Fig. 3C). Das zeigt an, dass eine verstärkte Blutplättchenadhäsion an Endothel zu einer Aktivierung von NF-κB *in vivo* führt.

### Vorübergehende Inkubation von HUVEC mit aktivierten Blutplättchen induziert eine Sekretion von MCP-1

Da signifikante Mengen von aktiviertem NF-κB in Bereichen beobachtet werden, die eine Blutplättchenadhäsion zeigen, wurden die Wirkungen aktivierter Blutplättchen auf kultivierte HUVEC untersucht. Zunächst wurde *in vitro* der Zeitverlauf der Blutplättchen-induzierten Endothelsekretion des Chemokins MCP-1 untersucht, das durch NF-κB reguliert wird (8, 9). Confluente Monoschichten von HUVEC wurden unbehandelt gelassen oder für 5 bis 300 Minuten mit α-Thrombin-aktivierten Blutplättchen, 100 pg/ml IL-1β oder 1,67 ng/ml TNF inkubiert. Blutplättchen oder Cytokine wurden anschließend von der endothelialen Monoschicht entfernt, und die Menge an MCP-1 im Überstand wurde nach weiteren vier Stunden Inkubation bestimmt (vorübergehende Co-Kultur).

Eine vorübergehende Inkubation von HUVEC mit aktivierten Blutplattchen für 30 Minuten ergab eine starke Erhöhung der MCP-1 Sekretion, die vergleichbar mit einer IL-1β-Stimulation (Fig. 4) ist, wobei der TNF-Effekt etwas schwächer war. Eine Inkubation von HUVEC mit ruhenden Blutplättchen induzierte keine signifikante Zunahme der MCP-1 Sekretion.

### Endotheliale NF-κB-Aktivierung und IκB-α/ε Proteolysemuster nach Inkubation mit Blutplättchen

Um den Mechanismus, mit dem aktivierte Blutplättchen die Expression von NF-κB-Targetgen-Produkten, wie MCP-1, induzieren, weiter zu beleuchten, wurden Zeitverlaufexperimente durchgeführt, um eine NF-κB-Aktivierung, die mit EMSA bestimmt wurde, zu beobachten. Aktivierte Blutplättchen induzierten eine starke und zeitabhängige Aktivierung von NF-κB in HUVEC, wobei maximale Effekte nach 60 Minuten beobachtet wurden (Fig. 5A). In denselben Kernextrakten wurde ebenfalls das Binden von Proteinen an Sp-1 Oligonukleotide, die als Loading-Control wirken (Fig. 5A), untersucht.

Anschließend wurde der Zeitrahmen und der Umfang der Blutplättchen-induzierten Proteolyse des IκB-Inhibitorproteins untersucht. Eine Inkubation von HUVEC mit aktivierten Blutplättchen sowie mit IL-1β führte zu einer signifikanten Proteolyse von IκB-α und IκB-ε (Fig. 5B und C). Obwohl die Proteolysemuster der IκB-Proteine im Anschluß an beide Stimuli ähnlich waren, wurden Unterschiede beobachtet: IκB-α wurde etwas schneller nach IL-1β-Einwirkung abgebaut, beginnend nach 15 Minuten nach der Inkubation, als im Falle einer Blutplättchen-Stimulation, bei der ein Abbau nach etwa 30 Minuten beobachtet wurde. Umgekehrt induzierten Blutplättchen eine frühere IκB-ε Proteolyse als IL-1β (signifikanter Effekt: 30 gegenüber 60 Minuten). In scharfem Kontrast dazu induzierte TNF einen weit schnelleren Abbau beider IκB-Proteine in HUVEC (Fig. 5D).

### Differentielle IKK-Komplex-Aktivierung in Endothelzellen nach Stimulierung mit Blutplättchen, IL-1β oder TNF

IκB-Proteine werden vor ihrem Abbau phosphoryliert, wobei dieser Prozess durch den IKK Komplex bewirkt wird. Um die Wirkung von Blutplättchen auf dieses Kinasesystem zu charakterisieren, wurden *in vitro* Kinase-Assays eingesetzt. HUVEC wurden mit aktivierten Blutplättchen, IL-1β oder TNF für die angegebenen Zeitdauern stimuliert bevor Zytosolextrakte hergestellt wurden. Eine Immunopräzipitation wurde entweder mit IKK-α oder IKK-β Antikörpern durchgeführt, und die Kinaseaktivität wurde bestimmt durch Messung des Phosphorylierungsstatus von GST-IκB-α. Eine Inkubation mit Blutplättchen induzierte eine merkliche Aktivierung des IKK-Komplexes mit maximalen Phosphorylierungsniveaus bei 30 Minuten, die innerhalb von 60 Minuten schnell auf Grundlinienwerte abfielen (Fig. 6A und D). Ähnliche Ergebnisse wurden erhalten, wenn IL-1β zu HUVEC zugesetzt wurde, obwohl Unterschiede beobachtet wurden bei einem Aktivierungspeak bei 45 Minuten, der für mindestens 90 Minuten beibehalten wurde (Fig. 6B und D). Wenn TNF eingesetzt wurde, um HUVEC zu stimulieren, setzte eine Aktivierung von IKK schnell innerhalb 2,5 Minuten ein und kehrte auf niedrige Werte innerhalb von 20 Minuten zurück (Fig. 6C und D). IKK-α und -β Niveaus blieben unter diesen Bedingungen konstant (Fig. 5B und C). Ähnliche Ergebnisse wurden unter Verwendung von ECV 304 Zellen erhalten.

### IKK-β und NIK sind an einer Blutplättchen-induzierten NF-κB Aktivierung beteiligt

Um die Rolle von IKK und NIK Proteinen in NF-κB betreffende Signalkaskaden zu untersuchen, wurde ECV 304 vor einer Stimulation mit Blutplättchen oder IL-1β mit Überexpressionsvektoren für den Wildtyp oder mutierte Flag-markierte Kinasen zusammen mit einem κB Luciferase-Reporter-Plasmid transformiert. Ein Western Blot unter Verwendung eines anti-Flag-Antikörpers bestätigte, dass die jeweiligen Proteine in dem vorliegenden System überexprimiert wurden und dass der Kontrollvektor ohne Insertion keine unspezifische Flag-Markierung zeigte. Zusätzliche Experimente zeigen, dass mit Flag markierte ausgefällte Proteine kinaseaktiv waren, wobei die mutierten Formen keine Aktiviät zeigten. Wenn NIK überexprimiert wurde, wurde eine dramatische Induktion des 3xκB.luci-Signals beobachtet, verglichen mit der CMV Kontrolle, das weiter erhöht wurde durch Inkubation der Zellen mit aktivierten Blutplättchen oder IL-1β (Fig. 7A und B, linke Spalten). Eine Transfektion von mutiertem NIK bewirkte eine dramatische Verminderung dieses Effekts mit sowohl dem vom Wildtyp induzierten Signal als auch der zusätzlichen Erhöhung durch die weitere Stimulierung. Eine Cotransformierung von Wildtyp IKK-α und -β zusammen hatte auch einen direkten Effekt auf das 3κB.luci-Plasmid, das signifikant verstärkt wurde durch Stimulation mit entweder Blutplättchen oder IL-1β (Fig. 7A und B, linke Spalte). Eine Überexpression der mutierten Formen von IKK-α und -β zusammen inhibierte sowohl die direkten als auch die Stimulusinduzierten Effekte, wobei das Blutplättchen-induzierte Signal fast vollständig verschwand, wobei ein schwächerer Effekt für IL-1β beobachtet wurde. Wenn die Kinasen einzeln exprimiert wurden anstatt in Kombination, wurde eine signifikante Inhibierung mit IKK-β_{KA} gefunden als Folge einer Inkubation mit Blutblättchen oder IL-1β (Fig. 7A und B, rechte Spalten). IKK-α_{KA} zeigte einen inhibitorischen Effekt nur für Blutplättchen, aber nicht für IL-1β-stimulierte Zellen.

Der Effekt einer Überexpression von mutiertem IKK-α und -β auf die Blutplättchen- oder die IL-1β-induzierte Promotor-abhängige Transkription wurde ebenfalls untersucht. ECV304 wurden mit Überexpressionsvektoren für Wildtyp oder mutierte Kinasen zusammen mit Luciferase-Reporter-Plasmiden, die MCP-1 oder VCAM-1 κB-tragende Promotor-Fragmente enthielten, transformiert. In diesem Fall ergab nur IKK-β_{KA}, aber nicht IKK-α_{KA}, eine signifikante Reduktion einer Blutplättchen- oder IL-1β-induzierten Transkription unter der Regulation dieser Promotoren (Fig. 7C).

### Eine IKK-β_{KA} Überexpression inhibiert eine Blutplattchen-induzierte Sekretion von MCP-1 in rAAV-transduzierten HUVEC

Um die Wirkung einer IKK-β_{KA} Überexpression auf Proteinniveau zu zeigen, wurden Wildtyp oder mutierte IKK-β Sequenzen in ein rekombinantes Adeno-assoziiertes Virussystem kloniert (13, 14). Die Untersuchungen wurden ursprünglich durchgeführt, um zu prüfen, ob rAAV primäre HUVEC-Kulturen transduzieren können. Subkonfluente Endothelial-Monoschichten wurden mit rAAV-GFP für 16 Stunden inkubiert, und nach weiteren 48 Stunden in Zellkultur wurde die Expression von GFP in infizierten HUVEC durch Fließzytometrie und Fluoreszenzmikroskopie analysiert (Fig. 8A und B). Es wurde gefunden, dass unter den angewendeten Bedingungen eine wesentliche Transduktion von HUVEC mit einer Effizienz von etwa 50% vorlag. Dies steht im vorteilhaften Gegensatz mit der Transfektionsrate von etwa 5 bis 10%, wie sie in HUVEC unter Verwendung herkömmlicher Transfektionsmethoden gefunden wird. Die Überexpression von IKK-β durch das rAAV-System wurde unter Verwendung einer Western-Analyse (Fig. 8C) bestätigt. Um die Effekte von IKK-β und IKK-β_{KA} Überexpression zu untersuchen, wurden HUVEC mit rAAV transduziert, das diese Proteine kodierte oder mit der Kontrolle rAAV-GFP. Nach der anfänglichen Inkubation, der eine weitere Kultivierung für 48 Stunden folgte, wurden die transduzierten Zellen mit aktivierten Blutplättchen oder IL-1β für 60 Minuten behandelt, und eine Sekretion von MCP-1 wurde nach 4 Stunden bestimmt. Zellen, die mit rAAV-IKK-β_{KA} transduziert waren, zeigten eine signifikante Abnahme der Blutplättchen- oder IL-1β-induzierten MCP-1 Sekretion, verglichen mit rAAV-GFP oder rAAV-IKK-β-behandelten Zellen (Fig. 8D). Diese Ergebnisse zeigen, dass das rAAV-System und die dadurch erhaltene hohe Transfektionseffizienz den direkten Nachweis über eine Bestimmung der Proteinkonzentration erlaubt, dass die dominant-negative Mutante IKK-β_{KA} stark die Expression des endothelialen Entzündungsgens MCP-1 inhibiert, die durch vorübergehende Wechselwirkung mit aktivierten Blutplättchen oder IL-1β induziert wird.

### Literatur:

1. Massberg et al. (1998) *Blood* 92, 507-512.
2. Massberg et al. (1999) *Blood* 94, 3829-3838.
3. Brand et al. (1996) *J Clin Invest* 97, 1715-1722.
4. Jaffe et al. (1973) *J Clin Invest* 52, 2745-2752.
5. Dickfeld et al. (2001) *Cardiovasc Res* 49, 189-199.
6. Page et al. (1999) *J Biol Chem* 274, 11611-11618.
7. Fischer et al. (1999) *J Biol Chem* 274, 24625-24632.
8. Ueda et al. (1997) *J Biol Chem* 272, 31092-31099.
9. Martin et al. (1997) *Eur J Immunol* 27, 1091-1097.
10. Gawaz et al. (1998) *Circulation* 98, 1164-1171.
11. Zolotukhin et al. (1999) *Gene Ther* 6, 973-985.
12. Grimm und Kleinschmidt (1999) *Hum Gene Ther* 10, 2445-2450
13. Hermens et al. (1999) *Hum Gene Ther* 10, 1885-1891.
14. Hauswirth et al. (2000) *Methods Enzymol* 316, 743-761

## Patentansprüche

1. Screeningverfahren zum Auffinden von Inhibitoren von IKK-β in Endothelzellen, wobei das Verfahren ein Kontaktieren eines potentiellen Inhibitors mit aktivierten Endothelzellen und eine Bestimmung einer Sekretion von MCP-1 aus den Endothelzellen umfasst.

2. Screeningverfahren nach Anspruch 1, wobei die Endothelzellen mit aktivierten Blutplättchen aktiviert werden.

3. Verwendung eines Inhibitors von IKK-β, der ausgewählt wird aus Sulindac, NEMO-binding domain peptide, 3-[(4-methylphenyl)-sulfonyl]-2-propennitril und 3-[(4-t-butylphenyl)-sulfonyl]-2-propennitril, zur Herstellung eines Medikaments zur
(a) Primärprophylaxe von Atherosklerose bei Patienten deren PROCAM-Wert größer oder gleich 50 beträgt, oder zur
(b) Sekundärprophylaxe nach einem kardiovaskulären Ereignis.

## Claims

1. A screening method for discovering inhibitors of IKK-β in endothelial cells, wherein the method comprises contacting a potential inhibitor with activated endothelial cells and determining secretion of MCP-1 from the endothelial cells.

2. A screening method according to claim 2, wherein the endothelial cells are activated by activated blood platelets.

3. The use of an inhibitor of IKK-β, selected from sulindac, NEMO-binding domain peptide, 3-[(4-methylphenyl)-sulfonyl]-2-propenenitrile and 3-[(4-t-butylphenyl)-sulfonyl]-2-propenenitrile, for the preparation of a medicament for
(a) primary prophylaxis of atherosclerosis in patients whose PROCAM value is greater than or equal to 50, or for
(b) secondary prophylaxis after a cardiovascular event.

## Revendications

1. Procédé de criblage pour détecter des inhibiteurs de la IKK-β dans des cellules de l'endothélium, lequel procédé comprend la mise en contact d'un inhibiteur potentiel avec des cellules activées de l'endothélium et la détermination d'une sécrétion de MCP-1 à partir des cellules de l'endothélium.

2. Procédé de criblage selon la revendication 1, dans lequel les cellules de l'endothélium sont activées par des plaquettes sanguines activées.

3. Utilisation d'un inhibiteur de IKK-β, choisi parmi le Sulindac, le peptide du domaine de liaison de NEMO, le 3-[(4-méthylphényl)-sulfonyl]-2-propènenitrile et le 3-[(4-t-butylphényl)-sulfonyl]-2-propènenitrile, pour la fabrication d'un médicament pour
(a) la prophylaxie primaire de l'athérosclérose chez des patients dont la valeur PROCAM est supérieure ou égale à 50, ou pour
(b) la prophylaxie secondaire après un accident cardiovasculaire.
